**Europäisches Patentamt**

**European Patent Office**

**Office européen des brevets**

(19)

(11) Publication number : **0 409 862 B1**

# EUROPEAN PATENT SPECIFICATION

(12)

(45) Date of publication of patent specification :
**11.11.92 Bulletin 92/46**

(51) Int. Cl.⁵ : **A61K 7/04,** A61K 7/48,
A61K 7/50, A61K 31/71

(21) Application number : **89904146.1**

(22) Date of filing : **10.03.89**

(86) International application number :
**PCT/GB89/00242**

(87) International publication number :
**WO 89/08442 21.09.89 Gazette 89/23**

(54) **TOPICAL PREPARATIONS.**

(30) Priority : **11.03.88 GB 8805814**

(43) Date of publication of application :
**30.01.91 Bulletin 91/05**

(45) Publication of the grant of the patent :
**11.11.92 Bulletin 92/46**

(84) Designated Contracting States :
**AT BE CH DE FR GB IT LI LU NL SE**

(56) References cited :
**WO-A-87/05806**
**FR-A- 2 566 661**
**US-A- 2 510 946**

(56) References cited :
**US-A- 3 821 370**
**Parfums, Cosmétiques, Arômes, no. 79, February/March 1988, (Paris, FR), H. Djelassi et al.: "L'ongle: griffe ou ornement",pages 47-61**

(73) Proprietor : **Seton Healthcare Group plc**
**Tubiton House Medlock Street**
**Oldham Lancashire OL1 3HS (GB)**

(72) Inventor : **MCROBERTS, Carine**
**27 Grant Street Cullen By Buckie**
**Banffshire AB5 2RS (GB)**

(74) Representative : **Quest, Barry et al**
**M'CAW & Co. 41-51 Royal Exchange Cross Street**
**Manchester M2 7BD (GB)**

**Description**

TECHNICAL FIELD

This invention relates to a topical preparation for use in treating fungal conditions particularly, but not exclusively, on human skin and nails.

BACKGROUND ART

Griseofulvin, a substance of general formula $C_{17}H_{17}C10_6$ (7 chloro - 4, 6 - dimethoxy coumaran - 3 - one - 2 - spiro - 1' - (2' - methoxy - 6' - methylcyclohex - 2' - en - 4' - one) which is produced by moulds of the genus <u>Penicillium</u>, is known to be an effective fungicidal agent.

Conventionally fungal conditions of the skin and nails are treated by oral administration of griseofulvin.

Oral administration for treatment of human skin and nails is not preferred. However, it has been found difficult to formulate satisfactory griseofulvin-containing topical preparations which are safe and effective. Griseofulvin is normally obtained as a fine powder which is essentially insoluble in water. The substance is soluble in various organic liquids such as alcohols (see for example WO-A-87/05806) but dissolved griseofulvin is not readily retained on the patients skin or nails within the fungal cells. In this respect, if adequate, sustained, penetration of the fungal cells with the fungicidal agent is not achieved prolonged treatment, perhaps extending over a period of several months, may be necessary to eradicate a fungal infection. It is believed that one reason for the problem which arises with griseofulvin may be that the substance in solution tends readily to migrate both into and out of the cells to be treated whereby it is difficult to achieve an adequate dwell time for effective treatment purposes.

Proposals have been made for incorporating in griseofulvin-containing topical preparations substances intended to facilitate retention in the skin. In this respect GB 1525120, GB 1538903 and U.S. 3932653 described griseofulvin-containing topical preparations incorporating 2 - pyrrolidone and N - alkyl - 2 - pyrrolidone. However, for effective treatment it is important that the preparation should have good migration into and retention within fungal cells, as well as skin cells. Also, it is important that all substances used in the preparation should have good skin compatibility in that there is little or no tendency for adverse skin reactions to occur.

FR-A-2566661 discloses topical preparations comprising dissolved griseofulvin. Eugenols (phenol-derivatives) are used as the solvent. US-A-3821370 discloses a phenol/resorcinol solvent system which is used for a salicylate-comprising topical composition.

An object of the present invention is to provide an improved skin-compatible topical preparation containing griseofulvin with which fungal infections can be treated in a particularly effective and expeditious manner.

DISCLOSURE OF THE INVENTION

According to the invention therefore there is provided a topical preparation for use in treating fungal conditions containing an active fungicidal agent comprising griseofulvin characterised in that the preparation also incorporates resorcinol and phenol, and the resorcinol, phenol and griseofulvin are present in the range of parts by weight 0.01 -2.4 resorcinol to 0.06 - 13.5 phenol to 2.5 griseofulvin.

With this preparation, when topically applied to a person's skin, it is believed that the resorcinol/phenol combination acts to weaken the S-H bonds in the epidermis and thereby facilitates the absorption of the active fungicidal agent of the preparation. Further, it is believed that the resorcinol/phenol combination aids in the breakdown of B, 1-4 Glucosamine in fungal wall cells so as to facilitate entry into the cells of the fungicidal agent. The effect is dramatic and it is found that unexpectedly rapid and effective treatment of fungicidal conditions is possible. Moreover, the resorcinol/phenol combination is especially skin-compatible whereby there is little or no tendency for inflammation or other adverse skin reactions to be caused by the presence of these substances.

Griseofulvin is an extremely active fungicidal agent in relation to a range of fungal infections. In order to promote further broad spectrum fungicidal activity a further fungicidal agent, particularly zinc undecenoate (i.e. zinc di (undec - 10 - enoate) is preferably also incorporated.

In this respect it is to be understood that a fungal condition may arise due to the presence of a range of different species whereby for effective treatment it is necessary to utilise fungicidal agents of adequate broad spectrum activity. Zinc undecenoate is a well known fungicidal agent for topical use. However when used with griseofulvin in the context of the present invention it is found that the resulting combination is unexpectedly exceptionally effective. That is the griseofulvin and zinc undecenoate provide an exceptionally broad spectrum of activity with each substance complementing the other to ensure that the preparation is extremely effective against very many of the fungal species encountered in practice. Moreover, exceptional compatibility is also achieved in that the desirable penetration and retention of the skin and fungal cells, and the desirable avoidance of adverse skin reactions, as discussed above, is not affected by the presence of the zinc salt.

Particularly with the abovementioned combination of zinc undecenoate and griseofulvin, the preparation has effective antibacterial as well as fungicidal activity and thereby helps to eliminate secondary skin infections whether of bacterial or fungal origin.

The above described ingredients of the preparation of the invention namely the griseofulvin, resorcinol, phenol and any zinc undecenoate, may be mixed with any suitable base, medium or solvent as desired and as appropriate to the intended physical form of the preparation.

Thus, in the case where the preparation is to be formulated as an ointment an appropriate emollient base will be incorporated.

Suitably, the emollient base should be a skin compatible composition whereby the preparation readily penetrates the epidermis and thereby facilitates the absorption of the active ingredients of the preparation. A particularly suitable base comprises a mixture of yellow soft paraffin and hydrous wool fat, preferably in equal parts (or approximately equal parts) by weight, such mixture being especially compatible with the skin. Wool fat resembles the subcutaneous secretions of human skin. By itself it is not readily absorbed into the tissues but when mixed with yellow soft paraffin it gives an emollient cream which readily penetrates the epidermis.

Whilst hydrous wool fat/yellow soft paraffin is preferred, any suitable ointment base may be used and the ointment may be of the oil-in-water or water-in-oil kind. Thus, combinations of paraffins, oils, fats and waxes may be selected in conventional manner as desired. The ointment base may contain vegetable oils, synthetic esters of fatty acids or animal fat such as lard, or wool fat or wool alcohols or macrogols, together with inert bases such as soft paraffin. Oil-in-water emulsifying agents such as emulsifying wax, cetrimide emulsifying wax or cetomacrogol emulsifying wax may be incorporated into anhydrous bases in the case where water is to be added.

The preparation of the invention may- be formulated as a cream, that is a semi-solid emulsion of oil-in-water or water-in-oil type. Emulsifying agents for aqueous creams include emulsifying waxes and sodium potassium, ammonium and triethanolamine soaps. Oily creams may be prepared with emulsifying agents such as wool fat, wool alcohols, beeswax, calcium soaps, sorbitan esters.

The preparation of the invention may be formulated as a paint or varnish i.e. an essentially liquid preparation which will adhere to the skin. Resinous substances such as benzoin, prepared storax or tolu balsam, in ethereal solutions are commonly employed as varnish bases. Collodium and amyl acetate are other examples.

The preparation of the invention may be formulated as a paste i.e. a semi-solid preparation. Pastes commonly consist of a high proportion of finely powdered solid matter mixed with soft or liquid paraffin or with a non-greasy base made with glycerin, mucilage or soap.

The preparation of the invention may be formulated as a lotion i.e. a liquid preparation containing substances such as alcohol and/or glycerin or other solvent and/or light oily substances.

Other kinds of preparations are also possible. It is to be understood that essentially it is the griseofulvin/phenol/resorcinol combination optionally with zinc undecenoate which is of importance. Any medium or solvent or base which is also incorporated is simply to assist in the application of the main ingredients and accordingly may be of any suitable nature. Thus it is even possible to use the main ingredients without any base, medium or solvent or with minimal quantities thereof. For example the preparation may be utilised as an aerosol spray or it may be absorbed onto an inert substrate such as a fabric dressing, a tissue wipe pad, insole etc. Especially in the case of an insole, the preparation of the invention may be absorbed onto a suitable material such as active charcoal which can receive exuded body fluids such as perspiration which can assist in release of the active ingredients of the preparation back to skin surface being treated.

Further, the preparation of the invention can be incorporated in a product whereby the product defines the application medium for the preparation and an added base is not therefore necessary. In particular the preparation may be incorporated in shampoo or bath foam or other soap-or-surfactant-containing cleansing product or the like. Thus, the preparation may be incorporated with spirit of soap or green soft soap or sodium lauryl benzene sulphonate, sodium stearate, acetylated lanolin, cetyl alcohol, sodium lauryl sulphate or the like.

The preparation may be formulated with a solvent to facilitate homogeneous mixture of the griseofulvin with the other ingredients. Any suitable solvent may be used and this may form part of the topical preparation as applied to a patients skin or nails, as would be the case if the preparation is in the form of an ointment or cream or lotion or the like, or it may be removed by evaporation after it has performed its dispersal function, as may be the case if the preparation is absorbed on a substrate such as active charcoal or if the preparation is used in the form of an aerosol spray. In a particularly preferred embodiment the solvent is an alcohol such as isopropyl alcohol, methanol, ethanol, or industrial spirits, especially isopropanol, such alcohols being especially advantageous because they dissolve griseofulvin and also zinc undecenoate and are skin compatible in that they do not have an undue irritant or astringent effect.

The preparation may contain further ingredients as desired and as appropriate. In general, having regard to the importance of ensuring that the active ingredients have good penetration of and retention in the skin and fungal cell walls and of ensuring that there is little likelihood of adverse skin reactions being caused, it is desirable to minimise additions of further ingredients. In particular solid or gum-like materials which may clog skin pores are generally undesirable as also are supplementary anti-bacterial or anti-fungal or other additives known to cause adverse skin reactions, although of course there may be circumstances in which such additives are appropriate.

Where the preparation is formulated with no significant content of ionic material the pH will generally tend to fall within an acceptable range, as also is the case when the preparation contains zinc undecenoate. That is, the pH tends to fall within the range of say 5 to 6. With preparations containing other ionic materials, for example where the preparation is incorporated in a shampoo or the like containing an ionic surfactant it may be necessary or desirable to incorporate a buffer to achieve a desired pH. In general a pH in the range 3 to 9, particularly 5 to 6 is desirable. Any suitable buffer may be used for example comprising an acid such as lactic acid, citric acid, tartaric acid, maleic acid, hydroxy-succinic acid with an acid salt such as any of sodium and potassium acid phosphate, sodium and potassium acid citrate, sodium and potassium acid tartrate.

Other non-therapeutic additives such as fragrances etc may be incorporated.

With regard to the proportions of the ingredients it is to be understood that it is the resorcinol: phenol: griseofulvin ratio which is important. In this respect the optimum ratio has been found to be 0.16:0.9:2.5. With this ratio there is sufficient phenol and resorcinol to achieve the desired penetration and retention of the griseofulvin. Significantly higher proportions of resorcinol and phenol are possible but do not appreciably increase penetration/retention. Significantly lower proportions of resorcinol/phenol may be possible but will reduce penetration/retention.

Thus, the ranges of the resorcinol and phenol are (in parts by weight)

0.01 - 2.4. resorcinol to
0.06 - 13.5 phenol to
2.5 griseofulvin.
The preferred ranges would be
.03 - 0.8 (particularly 0.1 - 0.2) resorcinol to
0.18 - 4.5 (particularly 0.8 - 1.0) phenol to
2.5 griseofulvin.
Within these ranges, although it is possible to use a greater proportion of resorcinol than phenol it is preferred that the phenol exceeds the resorcinol. Thus, the resorcinol:phenol ratio may be in the range 1:1.37 to 1:75 preferably 1:2 to 1:25 particularly 1:5 to 1:7.

With regard to the optional zinc undecenoate, this may be present in very small proportions relative to the griseofulvin and indeed it is an unexpected advantage of the invention that the high efficacy of the phenol/resorcinol penetration/retention medium is such that it is only necessary surprisingly to use a very small proportion of the zinc salt to supplement considerably the anti-fungal activity of the griseofulvin. Thus, the proportion of the zinc salt may be in the range (in parts by weight)

0.0007 to 0.15, preferably 0.002 to .05, particularly 0.008 to 0.012 relative to 2.5 parts by weight griseofulvin.

Where a solvent is used this may be present in a range (in parts by weight) of 0.06 to 13.5, preferably 0.2 to 4.5, particularly 0.7 to 1.1 relative to 2.5 parts by weight griseofulvin.

With regard to the proportion of griseofulvin in the preparation, this will depend on the nature of the preparation. If the preparation is an ointment or cream or the like have an appreciable emollient or similar base, the proportion of griseofulvin is preferably around 2.5% of the total weight of the preparation. The proportion may be in the range of 0.2% to 37.5%, preferably 0.5 to 17.5%, particularly 2% to 3%. In other preparations, other proportions of griseofulvin may be appropriate. Thus, where there is no base (e.g. because the preparation is absorbed on a substrate or is used in an aerosol spray) the proportion of griseofulvin may be around 50% of the preparation. The range may be 25% to 75% particularly 40% to 60%.

BEST MODE OF CARRYING OUT THE INVENTION

The invention will now be described further in the following Examples. All proportions are proportions by weight.

Example 1

The following ingredients were mixed in the stated proportions:

| | |
|---|---|
| Resorcinol | 0.160 |
| Liquefied phenol BP | 0.900 |
| Isopropyl alcohol | 0.918 |
| Grieseofulvin | 2.500 |
| Zinc undecenoate | 0.01 |
| Emollient base | 95.512 (i.e. to 100) |

The small quantities of resorcinol/phenol/isopropyl alcohol were obtained from a bulk stock solution. The base consisted of a mixture of yellow soft paraffin BP and hydrous wool fat BP in equal parts by weight.

The resulting mixture was a smooth cream. The cream was dermatologically acceptable, storage stable and effective against a wide range of fungal infections including the following:-

MICROSPORUM SPECIES (M)

M. audouinii
M. canis
M. cookei
M. distortum
M. equinum
M. ferrugineum
M. fulven
M. gallinae
M. gypsum
M. nanum
M. persicolor
M. praecox
M. racemosum
M. vanbrusseshemii

TRICHOPHYTON SPECIES (T)

T. concentricum
T. equinum
T. gourvillii
T. mentagraphytes
T. rubrum
T. schognleinii
T. simii
T. tonsuraus
T. verrucosum
T. violaceum
T. yaoudei
Epidermophyton floccosum
Candida albicans

The cream was also effective against the following conditions:-

Trophic ulcers, static ulcers, psoriasis, chilblains, varicose eczema and pruritis.

The efficacy of the cream was found to be such that the maximum treatment time was four weeks for nail infections of the kind which by conventional treatments can take 6-12 months to clear.

With the cream of Example 1, treatment times are greatly reduced and systemic side effects are almost completely absent.

In order to assess the efficacy and acceptability of the preparation of Example 1, clinical trials were undertaken as follows:

Patients suffering from fungal conditions were selected at random and were supplied with "Cream A" (i.e. the preparation of Example 1) in white 30g aluminium tubes. Each patient was asked to smear skin lesions with the cream daily. The extent and the severity of the lesions were recorded before and after treatment and photographs were taken. Patients were examined weekly and further skin and nail scrapings were taken for microscopic examination and culture in the laboratory. Several representative pieces of skin and nail were examined for the presence of fungal elements after digestion with 30% KOH solution. Sabouraud's dextrose agar supplemented with Cysteine 0.7% was innoculated with skin and nail scrapings and incubated at 37° Centi-

5

grade for 14 days to isolate the Dermatophytes and Yeasts. "Cream A" treatment was continued for 2 weeks after a clinical cure and later discontinued. At each attendance, patients were asked if they had any adverse reactions to the preparation they were using and whether they found the preparation acceptable. There was a six month follow-up of the patients after a cure was established.

RESULTS

6,230 patients with multiple diagnosis were selected for the study, irrespective of age, sex, severity of infection or health of the patient. The causitive organisms were:-
T. rubrum
T. mentagrophytes
E. floccosum
Microsporum
Candida albicans

## TABLE 1

| INFECTIVE ORGANISM | NO. OF PATIENTS WITH INFECTION |
|---|---|
| T. rubrum | 1020 |
| T. mentagrophytes | 1220 |
| E. floccosum | 800 |
| Microsporum | 220 |
| Candida albicans | 2970 |

## TABLE 2

| CAUSITIVE ORGANISM | SYSTEMATIC DISEASES |
|---|---|
| T. rubrum | 9% Peripheral vascular disease |
| T. mentagrophytes | 28% Anaemia (Iron) |
| E. floccosum | 1% Psorriasis |
| Microsporum | 5% Cardiovascular problems |

## TABLE 3

| CLINICAL DIAGNOSIS | TOTAL | CURED | NOT CURED | ADVERSE REACTION |
|---|---|---|---|---|
| T. rubrum | 1020 | 1020 | 0 | 0 |
| T. mentagrophytes | 1220 | 1220 | 0 | 0 |

| | | | | |
|---|---|---|---|---|
| E. floccosum | 800 | 800 | 0 | 0 |
| Microsporum | 220 | 220 | 0 | 0 |
| Candida albicans | 2970 | 2969 | 1 | 1 |

## COMMENTS

"Cream A" was effective on 99.98% of the patients treated. It was ineffective against 0. 02% (treatment discontinued due to adverse reaction). "Cream A" was found to be a broad-spectrum preparation which had fungicidal properties. There were no reports of relapse after discontinuation of "Cream A", after the follow-up period of 6 months. These findings are in conformity with the in vitro results of "Cream A", as explained hereinafter.

## DOUBLE BLIND CLINICAL TRIAL

The laboratory methods and selection of patients were the same as for the trials described above. The patients were advised that they may be given either a standard treatment or a placebo, or "Cream A". All of the patients had fungal infections of both feet.

The patients were randomised in blocks of 50. Within each block of 50 patients, there were approximately equal numbers with each of the common fungal infections listed in Table 1 above. Numbered tubes containing creams which were identical in appearance were prepared. Each patient received two tubes, one for the left, and one for the right foot, so that different treatments could be compared in the same patient. In all 244 patients were included.

Table 4 shows the results after 4 weeks of treatment, for each paired comparison.

### TABLE 4

**Numbers Clear or Not Clear after 4 weeks of treatment**

| | | Cream A | | Canesten | | Ecostat | | Tinefex | | Tineaderm | |
|---|---|---|---|---|---|---|---|---|---|---|---|
| | | C | N | C | N | C | N | C | N | C | N |
| Canesten | C: | 5 | 0 | | | | | | | | |
| | N: | 23 | 0 | | | | | | | | |
| Ecostat | C: | 1 | 0 | 5 | 0 | | | | | | |
| | N: | 10 | 0 | 0 | 24 | | | | | | |
| Tinefex | C: | 0 | 0 | 0 | 0 | 0 | 0 | | | | |
| | N: | 19 | 0 | 5 | 16 | 2 | 25 | | | | |
| Tineaderm | C: | 0 | 0 | 0 | 0 | 0 | 0 | 0 | 0 | | |
| | N: | 40 | 0 | 5 | 13 | 9 | 22 | 0 | 20 | | |
| Total | : | 98 | 0 | 20 | 76 | 17 | 81 | 0 | 87 | 0 | 109 |

Canesten, Ecostat, Tinefex, Tineaderm are Registered Trade Marks.

The table shows, for example, that in those patients who used "Cream A" on one foot, and Canesten on the other, there were five patients in whom both feet were clear at 4 weeks, and 23 in whom the foot treated with "Cream A" was clear, but not the other. No foot treated with "Cream A" did not clear.

Considering the individual infective agents involved the following results were obtained:

## TABLE 5

### Number of weeks until infection clear

#### CANDIDA ALBICANS

| Treatment | <4 | 4 | 6 | 8 | 10 | 10+ | Worse | Total |
|---|---|---|---|---|---|---|---|---|
| Cream A | 2 | 15 | - | - | - | - | - | 17 |
| Canesten | 2 | 18 | - | - | - | - | - | 20 |
| Ecostat | 1 | 16 | - | - | - | - | - | 17 |
| Tinefex | - | - | - | - | - | - | 16 | 16 |
| Tineaderm | - | - | - | - | - | - | 24 | 24 |

#### MICROSPORUM

| Treatment | <4 | 4 | 6 | 8 | 10 | 10+ | Worse | Total |
|---|---|---|---|---|---|---|---|---|
| Cream A | 2 | 17 | - | - | - | - | - | 19 |
| Canesten | - | - | - | - | - | - | 21 | 21 |
| Ecostat | - | - | - | - | - | - | 19 | 19 |
| Tinefex | - | - | - | - | - | 3 | 17 | 20 |
| Tineaderm | - | - | - | - | - | 3 | 16 | 19 |

#### T. RUBRUM

| Treatment | <4 | 4 | 6 | 8 | 10 | 10+ | Worse | Total |
|---|---|---|---|---|---|---|---|---|
| Cream A | 3 | 20 | - | - | - | - | - | 23 |
| Canesten | - | - | - | - | - | - | 11 | 11 |
| Ecostat | - | - | - | - | - | 1 | 25 | 26 |
| Tinefex | - | - | - | - | 1 | 20 | - | 21 |
| Tineaderm | - | - | - | - | - | 19 | - | 19 |

## T. MENTAGROPHYTES

| Treatment | <4 | 4 | 6 | 8 | 10 | 10+ | Worse | Total |
|---|---|---|---|---|---|---|---|---|
| Cream A | 2 | 17 | - | - | - | - | - | 19 |
| Canesten | - | - | - | - | - | - | 20 | 20 |
| Ecostat | - | - | - | - | - | - | 13 | 13 |
| Tinefex | - | - | - | - | 1 | 15 | 1 | 17 |
| Tineaderm | - | - | - | 1 | - | 24 | - | 25 |

## E. FLOCCOSUM

| Treatment | <4 | 4 | 6 | 8 | 10 | 10+ | Worse | Total |
|---|---|---|---|---|---|---|---|---|
| Cream A | 1 | 19 | - | - | - | - | - | 20 |
| Canesten | - | - | - | - | - | - | 24 | 24 |
| Ecostat | - | - | - | - | - | - | 23 | 23 |
| Tinefex | - | - | - | - | 1 | 2 | 10 | 13 |
| Tineaderm | - | - | - | - | - | 4 | 18 | 22 |

From these tables, it is plain that "Cream A", Canesten and Ecostat are about equally effective in dealing with Candida infections. On the other hand, Canestan and Ecostat are unsuccessful with other infections. Tinefex and Tineaderm clear infections with T. Rubrum, T. Mentagrophytes and E. Floccosum eventually, though not as fast as "Cream A". On the other hand, they fail with Candida infections. Only "Cream A" is consistently successful with Microsporum.

This means that if the diagnosis is unknown, "Cream A" has a clear advantage in both speed and efficacy.

No adverse reactions were observed in this trial.

The exceptional activity of "Cream A" can be explained in terms of the penetration of the skin and fungal cells and the retention of the active ingredients within such cells.

In this respect, the cell wall serves a number of important roles in fungi. It determines the characteristic shape of a cell, for when it is removed by enzymic treatments the resulting protoplast is always spherical. Consequently the way a fungus grows whether as a mycelium or as a yeast, and the range of structures it can produce are a direct result of its cell wall components and the ways these are incorporated into the wall during growth. Equally important, the wall acts as an interface between the protoplast and the environment. It protects the cell from osmotic lysis and perhaps metabolites of other organisms, but at the same time it must remain permeable to nutrients gases and enzymes. Also the wall acts as a binding site for these enzymes and it may have antigenic properties that mediate the interactions of fungi with other organisms.

Fungal walls contain a mixture of fibrillar and amorphous or matrix components. The fibrillar ones include chitin and cellulose both B(1-4) linked polymers and form a network of micofibrils which conferes rigidity on the wall. The amorphous components include proteins, mannans and B(1-3) and (1-3) linked glucans and are usually embedded within the fibrillar network.

Chitin is sufficiently common to be considered a characteristic wall component of fungi.

Mannans often replace chitin or glucans in the walls of yeasts.

With regard to the skin, the primary function of the epidermis is to generate a protective sheath, the stratum corneum that protects the organism from both desiccation and external assault. However, despite the stratum corneums acknowledged importance investigations of the cellular basis of its permeability has been hampered by extraordinary difficulties in its preservation for ultra structural study. Although the stratum corneum is very thin it is remarkably tough and resilient. It is highly impervious to water and most polar substances but lipid soluble substances depending on there chain length, branching, saturation and vehicle delivery system can penetrate the epidermis.

Epidermal lamellar bodies (Odland bodies, keratinosomes, membrane coating granules) are 0.1-0.3um ovoid organelles which are synthesised in stratum spinosum and stratum granulosum. They appear to be packaged in the smooth endoplasmic reticulum and reportedly contain polysaccharides, hydrolytic enzymes and lipids in the mid to upper stratum granulosum. They collect at the cell periphery and apex where their contents are expelled into the inter cellular spaces. While it is likely that the lamellar body may be involved in several epidermal functions. One of the most important may be the deposition of lipid rich intercellular materials which are necessary for the epidermal permeability barrier, following secretion the disc and dense material lie in discrete intercellular domains thus greatly expanding the intercellular spaces of upper stratum granulosum. Freeze fracture images of lamellar bodies show their contents primarily in cross fracture which suggests that they contain a predominance of polar materials.

Considering now the mode of action of "Cream A", griseofulvin preferentially distributes in the stratum corneum and enters the horny layers by diffusional forces being carried by the extracellular fluid and sweat through the epidermal cell layers. It reaches concentrations exceeding those of serum apparently due to a favourable partition into the horny layer cells. This partition is probably due to reversible protein binding and high solubility of the drug. If griseofulvin were to be applied topically without other constituents of "Cream A" there would be a rapid flux of griseofulvin in and out of the skin which would mean that no specific binding source would exist to hold griseofulvin on the epidermis, but the other constituents of "Cream A" give the binding stability required and as fungi attack the layers of skin the griseofulvin is able to successfully invade the fungal walls and cause a breakdown in B(1-3) and (1-3). "Cream A's" base resembles the sebaceous secretions of human skin therefore giving a vehicle which penetrates the layers of the epidermis thus facilitating the absorption of the fungicidal components of "Cream A". The resorcinol relieves itching. Phenol precipitates protein hence breakdown of fungal cell wall components therefore allowing penetration of griseofulvin into fungal hyphae. The griseofulvin causes a morphological change in the fungi i.e. the normal oval shape of the hyphae becomes spiral-like therefore increasing the surface area so the fungi can once again attacked by resorcinol and zinc undecenoate which in turn causes toxins which produce fungal death.

Although it is possible to postulate a mechanism for the action of "Cream A" it is to be understood that this is based on speculation and in fact the preparation is of surprising efficacy indicating that an unexpectedly dramatic effect is involved.

Further example formulations were prepared to assess the importance of the various ingredients, as follows:

Example 2 - as "Cream A" but with griseofulvin omitted.

Example 3 - as "Cream A" but with resorcinol omitted.

Example 4 - as "Cream A" but with Zinc Undecenoate omitted.

Example 5 - as "Cream A" but with phenol omitted.

The preparation of each of Examples 2 to 5 was tested *in vitro* against continuous stock cultures of the following kinds:

T. rubrum

T. mentagrophytes

E. floccosum

Microsporum

Candida

Spore counts were taken at 2 hour intervals.

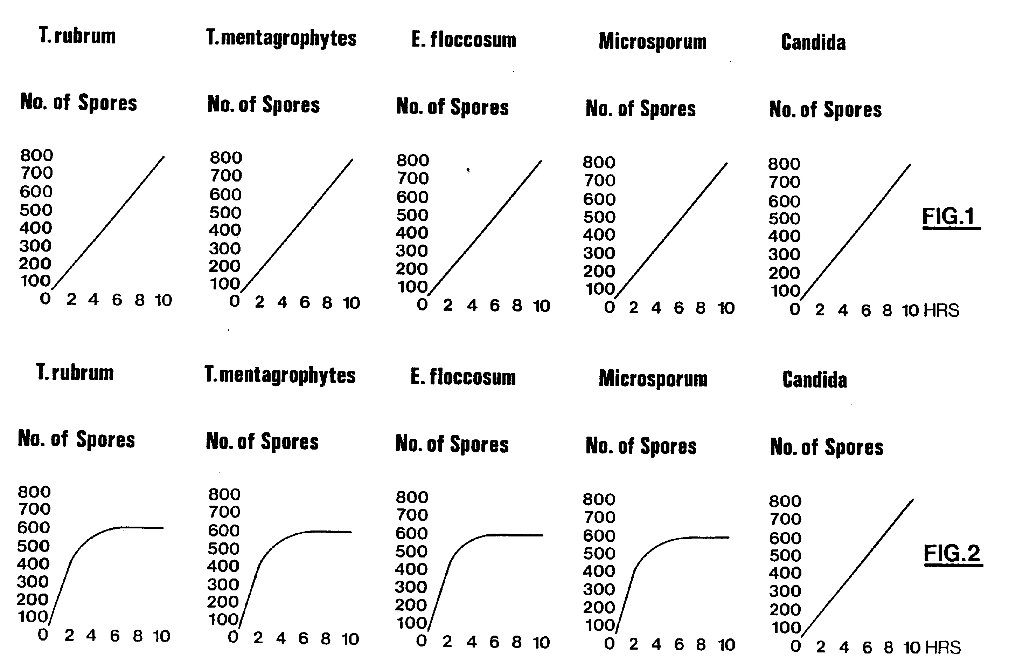

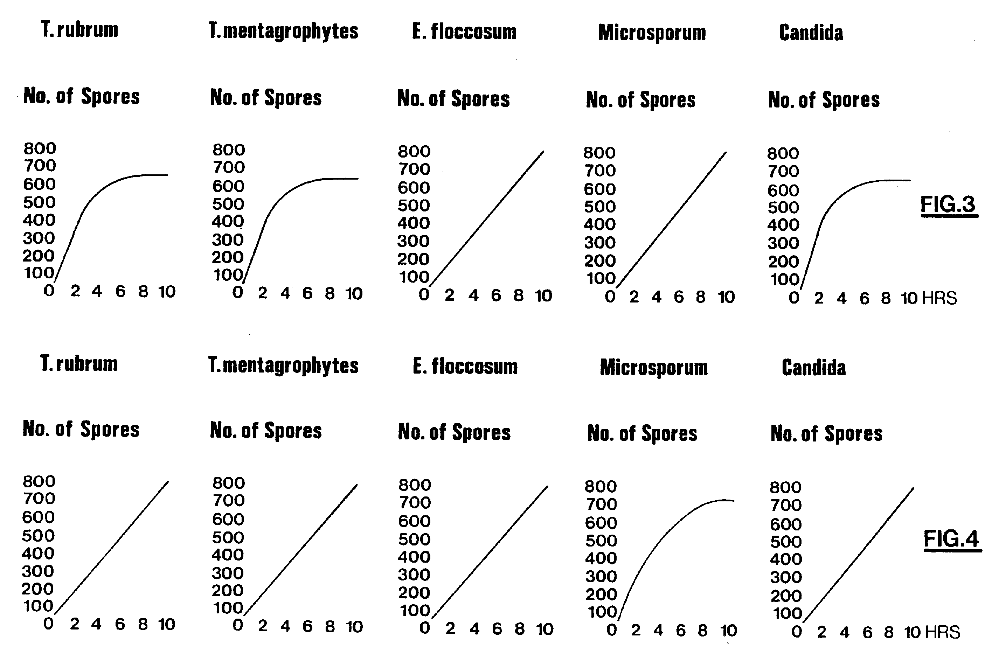

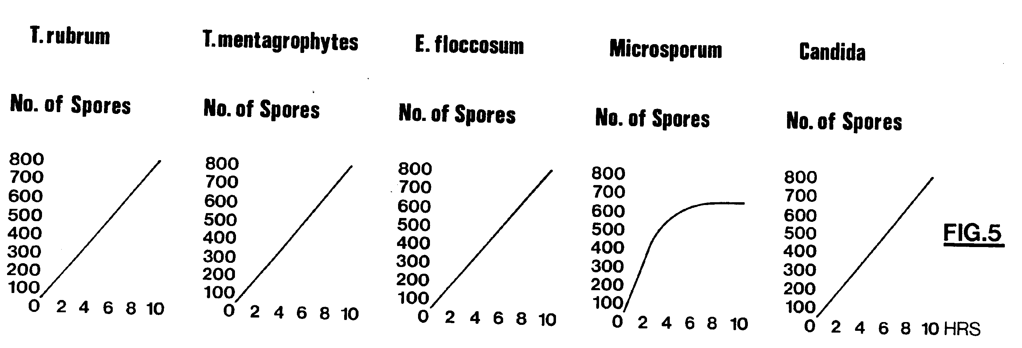

The results are shown in the graphs of Figs. 2 - 5. It will be seen that broad spectrum activity is appreciably adversely affected in Examples 2 - 5, showing the importance of utilising all of the four active ingredients in combination. Fig. 1 represents a control example using "Cream A" with all active ingredients omitted.

The *in vitro* tests were repeated increasing reach of the four active ingredients in turn by 1.5 times, in Examples 6 - 9. In each case the results were essentially the same as in Fig. 1 showing that increased proportions of ingredients are not of great value.

The *in vitro* tests were repeated decreasing each of the four active ingredients in turn by 2/3, in Examples 10 - 12. In each graphs similar to Fig. 1 but slightly less steep were obtained showing that reduction in active ingredients products a corresponding reduction in activity.

Example 13 A shampoo preparation was prepared by mixing the following ingredients:

Spirit Soap to 100

Resorcinol 0.198

Liquified Phenol BP 0.998
Isopropyl Alcohol 0.900
Griseofulvin 1.9
Zinc Undecenoate 0.05

Example 14 A bath foam preparation was prepared by mixing the following ingredients:
Sodium lauryl benzene sulphonate to 100
Resorcinol 0.195
Liquified Phenol BP 0.991
Isopropyl Alcohol 0.900
Griseofulvin 1.6
Zinc Undecenoate 0.06

Example 15 A paint (varnish) preparation was prepared by mixing the following ingredients:
Flexible Collodium to 100
Resorcinol 0.199
Liquified Phenol BP 0.987
Isopropyl Alcohol 0.898
Griseofulvin 1.0
Zinc Undecenoate 0.08

Example 16 A paint (varnish preparation was prepared by mixing the following ingredients:
Amyl Acetate to 100
Resorcinol 0.196
Liquified Phenol BP 0.989
Isopropyl Alcohol 0.899
Griseofulvin 2.1
Zinc Undecenoate 0.09

In each case, the preparation of Examples 13 to 16 were tested in vitro as described above and gave results corresponding to a combination of the graphs of Figs. 2 - 5 i.e. with all cultures showing a levelling off of spore counts after 2 - 4 hours.

The pH of the preparation of Example 1 was found to be in the range 5.2 to 5.6 which is acceptable for dermatological use. Similiarly acceptable pH values were obtained for the preparations of Examples 6 to 16, generally in the range 3 to 9 whereby buffering was not required.

**Claims**

1. A topical preparation for use in treatment fungal conditions containing an active fungicidal agent comprising griseofulvin characterised in that the preparation also incorporates resorcinol and phenol, and the resorcinol, phenol and griseofulvin are present in the range of parts by weight 0.01 - 2.4 resorcinol to 0.06 - 13.5 phenol to 2.5 griseofulvin.

2. A topical preparation according to Claim 1 further comprising zinc undecenoate.

3. A topical preparation according to Claim 1 or 2 further comprising an ointment base.

4. A topical preparation according to Claim 3 wherein the ointment base comprises yellow soft paraffin wax and hydrous wool fat.

5. A topical preparation according to any one of Claims 1 to 4 further comprising an alcohol solvent.

6. A topical preparation according to any one of Claims 1 to 5 wherein the range is 0.03 - 0.8 resorcinol to 0.18 - 4.5 phenol to 2.5 griseofulvin.

7. A topical preparation according to Claim 6 wherein the range is 0.1 - 0.2 resorcinol to 0.8 - 1.0 phenol to 2.5 griseofulvin.

8. A topical preparation according to any one of Claims 1 to 7 wherein the resorcinol:phenol ratio is in the range 1:1.37 to 1:75.

9. A topical preparation according to Claim 8 wherein the range is 1:2 to 1:25.

**10.** A topical preparation according to Claim 9 wherein the range is 1:5 to 1:7.

**11.** A topical preparation according to Claim 2 or any Claim dependent thereon wherein the proportion of zinc undecenoate is in parts by weight 0.0007 to 0.15 relative to 2.5 parts griseofulvin.

**12.** A topical preparation according to Claim 11 wherein the proportion is 0.002 to 0.05.

**13.** A topical preparation according to Claim 12 wherein the proportion is 0.008 to 0.012.

**14.** A topical preparation according to Claim 1 comprising, in parts by weight:

| | |
|---|---|
| resorcinol | 0. 16 |
| phenol | 0.9 |
| isopopanol | 0.918 |
| griseofulvin | 2.5 |
| zinc undecenoate | 0.01 |
| emollient base | to 100 |

**Patentansprüche**

**1.** Topisches Präparat zur Verwendung bei der Behandlung von Pilzzuständen, welches ein wirksames fungizides Mittel enthält, das Griseofulvin umfaßt, dadurch gekennzeichnet, daß das Präparat auch Resorcin und Phenol enthält und daß das Resorcin, das Phenol und das Griseofulvin in dem Bereich an Gewichtsteilen von 0,01 - 2,4 Resorcin zu 0,06 - 13,5 Phenol zu 2,5 Griseofulvin vorliegt.

**2.** Topisches Präparat nach Anspruch 1, welches weiter Zinkundecanoat umfaßt.

**3.** Topisches Präparat nach Anspruch 1 oder 2, welches weiter eine Salbenbasis umfaßt.

**4.** Topisches Präparat nach Anspruch 3, worin die Salbenbasis gelbes weiches Paraffinwachs und wasserhaltiges Wollfett umfaßt.

**5.** Topisches Präparat nach irgendeinem der Ansprüche 1 bis 4, welches weiter ein Alkohollösungsmittel umfaßt.

**6.** Topisches Präparat nach irgendeinem der Ansprüche 1 bis 5, worin der Bereich 0,03 bis 0,8 Resorcin zu 0,18 bis 4,5 Phenol zu 2,5 Griseofulvin ist.

**7.** Topisches Präparat nach Anspruch 6, worin der Bereich 0,1 bis 0,2 Resorcin zu 0,8 bis 1,0 Phenol zu 2,5 Griseofulvin ist.

**8.** Topisches Präparat nach irgendeinem der Ansprüche 1 bis 7, worin das Verhältnis Resorcin:Phenol im Bereich von 1:1,37 bis 1:75 liegt.

**9.** Topisches Präparat nach Anspruch 8, worin der Bereich 1:2 bis 1:25 ist.

**10.** Topisches Präparat nach Anspruch 9, worin der Bereich 1:5 bis 1:7 ist.

**11.** Topisches Präparat nach Anspruch 2 oder irgendeinem Anspruch, der von diesem abhängig ist, worin der Anteil an Zinkundecanoat 0,0007 bis 0,15 Gewichtsteile gegenüber 2,5 Teilen Griseofulvin beträgt.

**12.** Topisches Präparat nach Aspruch 11, worin der Anteil 0,002 bis 0,05 ist.

**13.** Topisches Präparat nach Anspruch 12, worin der Anteil 0,008 bis 0,012 ist.

**14.** Topisches Präparat nach Anspruch 1, umfassend in Gewichtsteilen:

| | |
|---|---|
| Resorcin | 0,16 |
| Phenol | 0,9 |
| Isopropanol | 0,918 |
| Griseofulvin | 2,5 |

| | |
|---|---|
| Zinkundecanoat | 0,01 |
| erweichender Grundstoff | auf 100 |

## Revendications

1. Préparation topique à utiliser dans le traitement d'affections fongiques, contenant un agent fongicide actif comprenant de la griséofulvine, caractérisée en ce que la préparation contient également du résorcinol et du phénol, et le résorcinol, le phénol et la griséofulvine sont présents dans les intervalles, en parties en poids, de 0,01 à 2,4 de résorcinol pour 0,06 à 13,5 de phénol pour 2,5 de griséofulvine.

2. Préparation topique selon la revendication 1, comprenant de plus de l'undécénoate de zinc.

3. Préparation topique selon la revendication 1 ou 2, comprenant de plus une base de pommade.

4. Préparation topique selon la revendication 3, dans laquelle la base de pommade comprend de la vaseline jaune et du suint hydraté.

5. Préparation topique selon l'une quelconque des revendications 1 à 4, comprenant de plus un alcool comme solvant.

6. Préparation topique selon l'une quelconque des revendications 1 à 5, dans laquelle les intervalles sont de 0,03 à 0,8 de résorcinol pour 0,18 à 4,5 de phénol pour 2,5 de griséofulvine.

7. Préparation topique selon la revendication 6, dans laquelle les intervalles sont de 0,1 à 0,2 de résorcinol pour 0,8 à 1,0 de phénol pour 2,5 de griséofulvine.

8. Préparation topique selon l'une quelconque des revendications 1 à 7, dans laquelle le rapport résorcinol: phénol se situe dans l'intervalle de 1:1,37 à 1:75.

9. Préparation topique selon la revendication 8, dans laquelle l'intervalle est de 1:2 à 1:25.

10. Préparation topique selon la revendication 9, dans laquelle l'intervalle est de 1:5 à 1:7.

11. Préparation topique selon la revendication 2 ou une quelconque revendication dépendante de celle-ci, dans laquelle la proportion d'undécénoate de zinc est, en parties en poids, de 0, 0007 à 0,15 pour 2,5 parties de griséofulvine.

12. Préparation topique selon la revendication 11, dans laquelle la proportion est de 0,002 à 0,05.

13. Préparation topique selon la revendication 12, dans laquelle la proportion est de 0,008 à 0,012.

14. Préparation topique selon la revendication 1, comprenant, en parties en poids :

| | |
|---|---|
| résorcinol | 0,16 |
| phénol | 0,9 |
| isopropanol | 0,918 |
| griséofulvine | 2,5 |
| undécénoate de zinc | 0,01 |
| base émolliente, jusqu'à | 100 |

**T.rubrum**　　**T.mentagrophytes**　　**E. floccosum**　　**Microsporum**　　**Candida**

No. of Spores　　No. of Spores　　No. of Spores　　No. of Spores　　No. of Spores

```
800                800                800                800                800
700                700                700                700                700
600                600                600                600                600
500                500                500                500                500         FIG.1
400                400                400                400                400
300                300                300                300                300
200                200                200                200                200
100                100                100                100                100
  0 2 4 6 8 10       0 2 4 6 8 10       0 2 4 6 8 10       0 2 4 6 8 10       0 2 4 6 8 10 HRS
```

**T.rubrum**　　**T.mentagrophytes**　　**E. floccosum**　　**Microsporum**　　**Candida**

No. of Spores　　No. of Spores　　No. of Spores　　No. of Spores　　No. of Spores

```
800                800                800                800                800
700                700                700                700                700
600                600                600                600                600
500                500                500                500                500         FIG.2
400                400                400                400                400
300                300                300                300                300
200                200                200                200                200
100                100                100                100                100
  0 2 4 6 8 10       0 2 4 6 8 10       0 2 4 6 8 10       0 2 4 6 8 10       0 2 4 6 8 10 HRS
```

EP 0 409 862 B1

**FIG.3**

T.rubrum — No. of Spores

T.mentagrophytes — No. of Spores

E. floccosum — No. of Spores

Microsporum — No. of Spores

Candida — No. of Spores

**FIG.4**

T.rubrum — No. of Spores

T.mentagrophytes — No. of Spores

E. floccosum — No. of Spores

Microsporum — No. of Spores

Candida — No. of Spores

FIG.5